Europäisches Patentamt

European Patent Office

Office européen des brevets

(19)

(11) Publication number: **0 125 125**
A2

(12) # EUROPEAN PATENT APPLICATION

(21) Application number: **84303035.4**

(22) Date of filing: **04.05.84**

(51) Int. Cl.³: **C 12 P 1/00**
C 12 N 5/00, A 61 K 35/12
//C12R1/91

(30) Priority: **06.05.83 JP 78300/83**

(43) Date of publication of application:
**14.11.84 Bulletin 84/46**

(84) Designated Contracting States:
**CH DE FR GB LI NL**

(71) Applicant: **KOKEN LTD.**
**No. 7, Yonban-cho**
**Chiyoda-ku Tokyo(JP)**

(71) Applicant: **Tajima, Tomoyuki**
**5-15, Yawata 6-chome**
**Ichikawa-shi Chiba-ken(JP)**

(71) Applicant: **Nagasu, Youichiro**
**No. 9-4, Chuo 3-chome**
**Yamato-shi Kanagawa-ken(JP)**

(72) Inventor: **Tajima, Tomoyuki**
**No. 5-15, Yawata 6-chome**
**Ichikawa-shi Chiba-ken(JP)**

(74) Representative: **Deans, Michael John Percy et al,**
**Lloyd Wise, Tregear & CO. Norman House 105-109**
**Strand**
**London WC2R OAE(GB)**

(54) An agent for inhibiting the proliferation of malignant tumour cells and a process for the preparation thereof.

(57) Agents for inhibiting the proliferation of malignant tumour cells are described together with processes for their preparation. Malignant tumour cells are cultivated in a culture medium for a period before being removed therefrom. By means of fractional filtration from the culture medium, that fraction having a molecular weight of not less than 500 is separated. Results show that this fraction has a significant inhibiting effect against proliferation of malignant tumour cells.

EP 0 125 125 A2

## AN AGENT FOR INHIBITING THE PROLIFERATION OF MALIGNANT TUMOUR CELLS AND A PROCESS FOR THE PREPARATION THEREOF

### Description

The present invention relates to agents for inhibiting the proliferation of malignant tumour cells and to processes for the preparation thereof.

In accordance with a first aspect of the present invention, there is provided a process for preparing an agent for inhibiting the proliferation of malignant tumour cells, characterised in that it comprises the steps of: cultivating malignant tumour cells in a culture medium; removing the malignant tumour cells from said culture medium; and separating from said culture medium by means of fractional filtration that fraction having a molecular weight of not less than 500.

According to a second and alternative aspect of the present invention, there is provided an agent for inhibiting the proliferation of malignant tumour cells, characterised in that it has a molecular weight of not less than 500 and in that it is derived by cultivating malignant tumour cells in a culture medium, removing the malignant tumour cells from said culture medium and separating the said agent by fractional filtration.

The malignant tumour cells are suitably cultured in an extraction medium at a temperature between $30^{o}C$ and $37^{o}C$. The fraction with a molecular weight not less than 500 is suitably separated by filtration through a molecular sieve, for example a membrane filter adapted to fractionate at a molecular weight of 500, such as a UM05 filter or a YA05 filter made by Amicon and adapted to separate the material being filtered into that fraction having a molecular weight not less than 500 and that fraction having a molecular weight of less than 500.

As will become clear from the description below, our experimental work on such fractions has shown that there is a markedly more apparent inhibiting activity against the proliferation of malignant tumour cells using the fraction having a molecular weight of not less than 500 as compared with the fraction having a molecular weight of less than 500. This same high inhibiting activity was apparent even when the said fraction was substantially diluted.

We have not found agents prepared in accordance with the present invention for inhibiting the proliferation of malignant tumour cells to show side effects of the nature and magnitude which commonly occur with conventional antineoplastic agents.

We have found that the process of the invention may conveniently be carried out by transplanting malignant tumour cells incubated in a growth culture medium into a second or extraction culture medium, cultivating the incubated cells in the said extraction culture medium, removing the tumour cells and subjecting the culture medium to ultrafiltration by passing the said medium through an ultrafiltration membrane capable of fractionating at a molecular weight of 500 so as to separate that fraction having a molecular weight of not less than 500.

The invention is hereinafter more particularly described by way of example only by reference to our experimental procedure.

We employed the following experimental material:

(i) an established cell line designated HRC originating from human renal carcinoma cells; and

(ii) an extraction culture medium comprising Basal Medium Eagle (hereinafter referred to as BME) for diploid cells supplemented with 10% new born calf serum.

The following cultivation method was employed:
In excess of $2 \times 10^7$ cells of an established HRC
cell line were placed with 50 ml of BME in a culture
bottle, the bottom surface area of which was 150 $cm^2$.
Cultivation was carried out for a period of one week.
Thereafter, the culture medium was subjected to
ultrafiltration by passing through an ultrafiltration
membrane capable of fractionating at a molecular
weight of 500.  Examples of such membrane which we
have used include a UM05 filter or a YA05 filter
made by Amicon.  The result is separation into a
first fraction having a molecular weight of not less
than 500 and a second fraction having a molecular
weight of less than 500.

The following assay method was employed to
establish inhibiting effect:
An amount of $10^4$ cells of an established HRC cell
line were inoculated in a 15 mm in diameter plastic
dish, and were cultivated in BME supplemented with
10% Bovine serum therein for 24 weeks.
First and second fractions, hereinafter referred to
as "fr.1" and "fr.2", and being the fractions having
a molecular weight respectively of not less than 500
and of less than 500 were produced as previously
described.
To the 24 week culture described above used for the
purpose of this assay, was added in one case the
fraction fr.1, in a second case the fraction fr.2
and in a third case the whole without fractionation.
To the culture medium in each case was added amino
acids, vitamins and glucose in the same amount as
the first BME was added, and the culture medium
replaced to its original amount.  The respective
cultures were then incubated at 37$^o$C under conditions
of 5% $CO_2$ and 100% humidity for six days.  The extent
to which the HRC cell line had proliferated in the
three cases was then studied.

Experimental results:

Table I set out hereinbelow shows the inhibiting rate against the proliferation of malignant tumour cells which we found in the three cases. Proliferation-inhibiting figures were calculated in each case by subtracting the surviving numbers of cells from the numbers originally present. The proliferation-inhibiting rate was then calculated as a percentage of the proliferation-inhibiting figure exhibited by the culture medium containing the fraction having a molecular weight of not less than 500.

Results are set out in Table I below. Comparative results are provided where the inhibiting inoculant was diluted by 2.

It will be apparent that the fraction fr.1 had a remarkably enhanced proliferating effect as compared either with the fraction fr.2 or with the whole unfractionated. The effect was even more noticeable on dilution.

The experiment was repeated a second time as indicated by the second lot number. The clear correspondence between the two sets of results shows that the effect is reproducable.

Table I

| Lot Nos. | Fraction | Proliferation-inhibiting rates | |
| --- | --- | --- | --- |
| | | Diluted X 1 | Diluted X 2 |
| 830112 | Whole | 82% | 28% |
| | fr. 1 | 100% | 94% |
| | fr. 2 | 32% | 6% |
| 74-1 | Whole | 62% | 11% |
| | fr. 1 | 100% | 97% |
| | fr. 2 | 14% | 0% |

CLAIMS

1.   A process for preparing an agent for inhibiting the proliferation of malignant tumour cells, characterised in that it comprises the steps of:  cultivating malignant tumour cells in a culture medium; removing the malignant tumour cells from said culture medium; and separating from said culture medium by means of fractional filtration that fraction having a molecular weight of not less than 500.

2.   A process according to Claim 1, further characterised in that the cultured malignant tumour cells are derived from an established cell line originating from a human renal carcinoma.

3.   A process according to Claim 1 or Claim 2, further characterised in that the culture medium comprises Basal Medium Eagle not containing serum.

4.   An agent for inhibiting the proliferation of malignant tumour cells whenever produced by a process according to any preceding claim.

5.   An agent for inhibiting the proliferation of malignant tumour cells, characterised in that it has a molecular weight of not less than 500 and in that it is derived by cultivating malignant tumour cells in a culture medium, removing the malignant tumour cells from said culture medium and separating the said agent by fractional filtration.

6.   The use of an agent according to Claim 4 or 5 for the treatment of the human or animal body by inhibiting the proliferation of malignant tumour cells.